# EUROPEAN PATENT APPLICATION

(11) **EP 4 603 121 A1**
(43) Date of publication of application: **20.08.2025**
(21) Application number: 24305240.4
(22) Date of filing: 13.02.2024
(51) Int. Cl.: A61M 5/315

(54) **STOPPER WITH DUAL RIB CONFIGURATION**

(71) Applicant: Becton Dickinson France, 38800 Le Pont-de-Claix (FR)
(72) Inventor: EUVRARD, Nicolas, London SW17 0JF (GB)
(74) Representative: Germain Maureau

(57) **Abstract**

A stopper including a main body defining a proximal end, a distal end, and a sidewall extending between the proximal end and the distal end; a first plurality of ribs adjacent the proximal end, the first plurality of ribs including a first rib extending radially outward around a perimeter of the main body, and a second rib extending radially outward around a perimeter of the main body, the second rib distally spaced from the first rib between 0.5 mm and 2 mm; and a second plurality of ribs adjacent the distal end, the second plurality of ribs including a third rib extending radially outward around a perimeter of the main body, and a fourth rib extending radially outward around a perimeter of the main body, the fourth rib distally spaced from the third rib between 0.5 mm and 2 mm.

## Description

### FIELD OF THE INVENTION

The present disclosure relates generally to stoppers for injection devices, and in particular, stoppers with improved resistance to contaminant entry into the injection device.

### BACKGROUND OF THE INVENTION

Prefilled injection devices are commonly-used containers to deliver drugs or vaccines to patients and include syringes, cartridges, and autoinjectors or the like. Such devices usually include a plunger attached to a stopper movably positioned within a container, such a syringe barrel, which is filled with a pharmaceutical composition in order to provide healthcare practitioners with a ready-to-use injection device for patients. Such injection devices may be exposed to various ambient pressures and temperatures when moved from a point of manufacturing, to storage, and/or to final use in a healthcare setting. For example, such devices may be shipped via one or more air courier routes, thus experiencing air pressure fluctuations. In addition, the devices may be stored or moved in and out of temperature controlled settings, resulting in temperature fluctuations. Such changes in ambient temperature and pressure can cause the stopper to move relative to the syringe barrel or other portion device, which can introduce contaminants into or around the stopper, and thus into an interior of the device.

The present disclosure provides systems, devices, and methods of use thereof that prevent or reduce the introduction of contaminants into a device due to stopper movement during transit and/or storage.

### SUMMARY OF THE INVENTION

The present disclosure provides a stopper adapted for use within a syringe barrel, the stopper comprising a main body defining a proximal end, a distal end, and a sidewall extending between the proximal end and the distal end; a first plurality of ribs adjacent the proximal end, the first plurality of ribs including a first rib extending radially outward around a perimeter of the main body, and a second rib extending radially outward around a perimeter of the main body, the second rib distally spaced from the first rib between 0.5 mm and 2 mm; and a second plurality of ribs adjacent the distal end, the second plurality of ribs including a third rib extending radially outward around a perimeter of the main body, and a fourth rib extending radially outward around a perimeter of the main body, the fourth rib distally spaced from the third rib between 0.5 mm and 2 mm.

In some non-limiting embodiments or aspects, the proximal end may be adapted to connect to a plunger rod.

In some non-limiting embodiments or aspects, the first rib may have a length between 0.5 mm and 1.5 mm.

In some non-limiting embodiments or aspects, the second rib may have a length between 0.5 mm and 1.5 mm.

In some non-limiting embodiments or aspects, the third rib may be distally spaced from the second rib between 2 mm and 4 mm.

In some non-limiting embodiments or aspects, the third rib may have a length between 0.5 mm and 1.5 mm.

In some non-limiting embodiments or aspects, the fourth rib may have a length between 0.5 mm and 1.5 mm.

In some non-limiting embodiments or aspects, each of the first rib and fourth rib may have a length greater than a length of either of the second rib or third rib.

In some non-limiting embodiments or aspects, the second plurality of ribs may be distally spaced from the first plurality ribs between 2 mm and 4 mm.

In some non-limiting embodiments or aspects, the second plurality of ribs may be distally spaced from the first plurality ribs a distance that is 2x to 8x a distance between the first and second ribs.

In some non-limiting embodiments or aspects, the present disclosure provides an injection device, comprising: a syringe barrel; and a stopper movably positioned within the syringe barrel, the stopper including: a main body defining a proximal end, a distal end, and a sidewall extending between the proximal end and the distal end; a first plurality of ribs adjacent the proximal end; and a second plurality of ribs adjacent the distal end, wherein the second plurality of ribs is distally spaced from the first plurality ribs between 2 mm and 4 mm.

In some non-limiting embodiments or aspects, at least one rib of the first plurality of ribs may define an interference level with respect to the syringe barrel between 3% and 15%.

In some non-limiting embodiments or aspects, at least one rib of the second plurality of ribs may define an interference level with respect to the syringe barrel between 3% and 15%.

In some non-limiting embodiments or aspects, each rib of the first plurality of ribs may have substantially the same length.

In some non-limiting embodiments or aspects, each rib of the second plurality of ribs may have substantially the same length.

In some non-limiting embodiments or aspects, at least one rib of the first plurality of ribs may have a larger length than at least one other rib of the first plurality of ribs.

In some non-limiting embodiments or aspects, at least one rib of the second plurality of ribs may have a larger length than at least one other rib of the second plurality of ribs.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings, wherein:
FIG. 1 is a side cross-sectional view of an example of a medical device constructed in accordance with the principles of the present disclosure;
FIG. 2 is a perspective view of an example of a stopper of the medical device of FIG. 1; and
FIG. 3 is a side view of the stopper of FIG. 2.

### DETAILED DESCRIPTION OF THE INVENTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As used herein, the singular form of "a", "an", and "the" include plural referents unless the context clearly dictates otherwise.

Spatial or directional terms, such as "left", "right", "inner", "outer", "above", "below", and the like, relate to the embodiments or aspects as shown in the drawing figures and are not to be considered as limiting as the embodiments or aspects can assume various alternative orientations.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

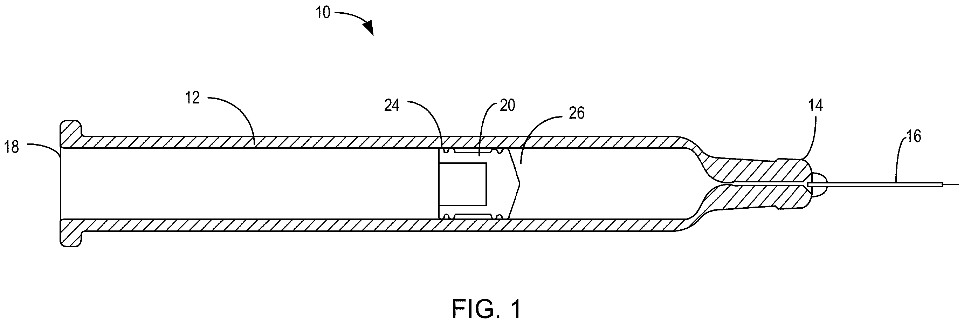

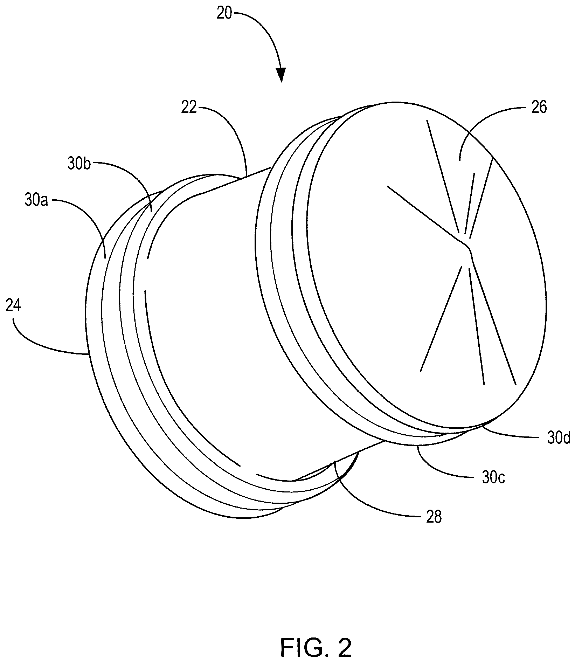

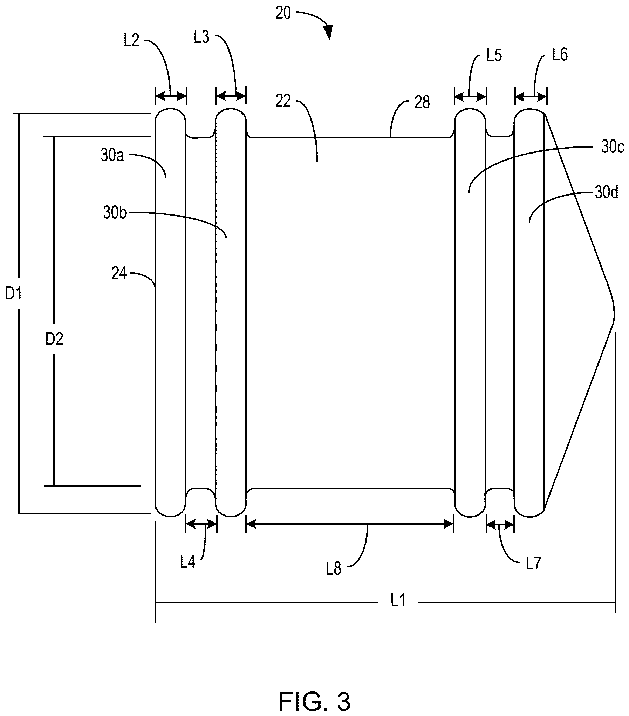

The present disclosure provides systems, devices, and methods of use thereof that prevent or reduce the introduction of contaminants into a device due to stopper movement during transit and/or storage. Referring now to the figures in which like reference designations refer to like elements, examples of a medical device 10 and aspects and components thereof are shown in FIGS. 1-3.

Reference is now made to FIG. 1, which shows a cross-sectional side view of an example of the medical device 10, which may generally include a syringe. The medical device 10 may define or include a syringe barrel 12 or other container defining or including a distal end 14. The distal end 14 may include an outlet opening coupled to a needle 16 and/or including a mechanism for attachment of a separate medical device (such as a catheter), for example, through a luer lock or other engageable interface. The syringe barrel 12 may include or define a proximal end 18 for receiving a stopper 20 movably positionable within the syringe barrel 12. The stopper 20 may be adapted or configured to operate with a plunger (not shown) attached to the stopper 20 to controllably actuate movement within the syringe barrel 12 to introduce or expel contents into or out of the syringe barrel 12.

The stopper 20 may define or include a main body 22 defining a proximal end 24, a distal end 26, and a sidewall 28 extending between the proximal end 24 and the distal end 26. The main body 22 may define an overall length L1 extending from the proximal end 24 to the distal end 26. The stopper 20 may include or define a substantially cylindrical shape. The proximal end 24 may be adapted or configured to receive or attach to a plunger, e.g., for example by defining a cavity or opening therein, one or more threads, or the like to facilitate an attachment. The distal end 26 of the stopper 20 may include an angled or contoured surface adapted or configured to conform to a curvature of the syringe barrel 12 to reduce or minimize dead space within the syringe barrel 12. For example, the stopper 20 may include a distal end including or defining a frustoconical shape and/or a substantially triangular side profile.

The stopper 20 may be constructed from one or more compressible materials or elastomers including but not limited to butyl rubber, styrene butadiene rubber, poly isoprene rubber, liquid silicone rubber, thermoplastic elastomer (TPE), and the like. Optionally, one or more portions of the stopper 20, such as the distal end 26 for example, may include a coating that acts as a barrier between the stopper 20 and the pharmaceutical or chemical composition that occupies the syringe barrel 12. The coating may reduce extraction and/or leaching of substances from the stopper material into the pharmaceutical or chemical composition, and vice versa. Such coatings may include, for example, silicone oil, PTFE, ETFE, liquid silicone rubber, and the like.

The stopper 20 may include or define one or more ribs extending radially outward around a perimeter of the main body 22 that are sized, shaped, and/or positioned about the main body 22 to form a seal with an interior of the syringe barrel 12 and to reduce the likelihood of contaminant entry and into the syringe barrel 12 and/or interaction with a substance or fluid within the syringe barrel 12. For example, the stopper 20 may include a first plurality of ribs at or adjacent to the proximal end 24 of the main body 22. The first plurality of ribs may include, for example, a first rib 30a and a second rib 30b longitudinally spaced from the first rib 30a in a distal direction. The first rib 30a may include or define a proximal-most surface or radial edge of the main body 22. The first rib 30a may define a length L2, the second rib 30b may define a length L3, and the distance between opposing faces of the first and second ribs 30a, 30b may define a length L4.

The stopper 20 may include a second plurality of ribs at or adjacent to the distal end 26 of the main body 22. The second plurality of ribs may include, for example, a third rib 30c and a fourth rib 30d longitudinally spaced from the third rib 30c in a distal direction. The fourth rib 30d may include or define a distal-most radial edge of the main body 22. The third rib 30c may define a length L5, the fourth rib 30d may define a length L6, and the distance between opposing faces of the third and fourth ribs 30c, 30d may define a length L7.

In one example, the lengths of all of the ribs 30a-30d may be substantially equal to each other. For example, the length for each rib may be between 0.5 mm and 1.5 mm (as measured prior to insertion into the syringe barrel 12). In another example, the lengths L2, L6 of the first rib 30 and the fourth rib 30d may be substantially equal to each other, and larger than the lengths L3, L5 of the second rib 30b and the third rib 30c. The larger lengths of the first and fourth ribs 30a, 30d can reduce the likelihood of contaminant ingress and intermingling with the contents of the syringe barrel 12, as described herein.

The ribs may be spaced from each other to provide an effective measure of redundancy and containment of the ingress of contaminants and/or egress of the contents of a syringe, as described herein. For example, the first rib 30a may be spaced a distance L4 from the second rib 30b such that the second rib can effectively limit the movement of a contaminant that passes by or through the first rib 30a. Similarly, the third rib 30c may be spaced a distance L7 from the fourth rib 30d such that the fourth rib 30d can effectively limit the movement of a contaminant that passes by or through the third rib 30c. The distance L7 may also enable the third rib 30c to sufficiently contain a leak or egress of the contents of the syringe that passes by or through the fourth rib 30d. The second rib 30b and the third rib 30c may be separated by a distance L8 that is larger than the distances L4, L7 to further reduce the likelihood that a contaminant or leaked contents can traverse the multitude of ribs of the stopper 20. In one non-limiting example, the distance L8 may be between 2x and 8x the distance L4 or L7.

In one non-limiting example, the distance L4 between the first rib 30a and the second rib 30b may be between 0.5 mm and 2 mm. In one non-limiting example, the distance L7 between the third rib 30c and the fourth rib 30d may be between 0.5 mm and 2 mm. In one non-limiting example, the distance L8 between the second rib 30b and the third rib 30c may be between 2 mm and 4 mm.

The rib 30a-30d may each define or include a diameter D1 that is wider or greater than a diameter D2 of the sidewall 28 of the main body 22 of the stopper 20. In one example, the diameter of all of the ribs 30a-30d may be substantially equal to each other.

The ribs 30a-30d may be sized and shaped to provide a desired interference level with the syringe barrel 12 to further reduce contaminant ingress and preserve overall container closure integrity. In particular, contact pressure between a rib of the stopper 20 and an interior surface of the syringe barrel 12 is affected in part by an interference level calculated as follows: Interference = (rib outer diameter - inner diameter of syringe barrel)/(rib outer diameter). In one example, each of the ribs 30a-30d provides an interference level between 3% and 15%. The ribs 30a-30 may each provide substantially the same interference level as each of the other ribs.

The configuration and features of the stopper 20 substantially limit or prevent movement of the stopper 20 within the syringe barrel 12 during exposure to temperature and/or pressure fluctuations which could otherwise introduce contaminants into the syringe barrel 12. For example, the proximity of the first rib 30a to the second rib 30b (e.g., the distance L4) provides as measure of redundancy in the event that the environmental changes causes the first rib 30a to move sufficiently to break the seal between the first rib 30a and the syringe barrel 12. In such an example, if the syringe barrel 16 has a contaminant just outside the proximal end 24 of the stopper 20 on or about an edge of the first rib 30a, movement of the stopper 20 could cause the contaminant to cross the first rib 30a. The second rib 30b in close proximity to the first rib 30a provides a redundant seal to prevent further ingress of the contaminant into the syringe barrel 12. In similar regard, the proximity of third rib 30c to the fourth rib 30d (e.g., the distance L7) may also provide a measure of redundancy to effectively seal or contain a leak of the contents of the syringe in the event that the effectiveness of the seal of fourth rib 30d is compromised.

If the amplitude of movement of the stopper 20 resulting from environmental changes is sufficient to cause contact or travel of a contaminant to and/or distally of the second rib 30b, then the spacing L8 between the second rib 30b and third rib 30c provides another encapsulating or sealing region to prevent introduction of the contaminant into the contents of the syringe barrel 12. The spacing L8 may also provide an extended region to contain a leak of the contents of the syringe should the ribs 30c and or 30d be compromised.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A stopper adapted for use within a syringe barrel, the stopper comprising:
a main body defining a proximal end, a distal end, and a sidewall extending between the proximal end and the distal end;
a first plurality of ribs adjacent the proximal end, the first plurality of ribs including a first rib extending radially outward around a perimeter of the main body, and a second rib extending radially outward around a perimeter of the main body, the second rib distally spaced from the first rib between 0.5 mm and 2 mm; and
a second plurality of ribs adjacent the distal end, the second plurality of ribs including a third rib extending radially outward around a perimeter of the main body, and a fourth rib extending radially outward around a perimeter of the main body, the fourth rib distally spaced from the third rib between 0.5 mm and 2 mm.

2. The stopper of claim 1, wherein the proximal end is adapted to connect to a plunger rod.

3. The stopper of any of the preceding claims, wherein the first rib has a length between 0.5 mm and 1.5 mm.

4. The stopper of any of the preceding claims, wherein the second rib has a length between 0.5 mm and 1.5 mm.

5. The stopper of any of the preceding claims 1, wherein the third rib is distally spaced from the second rib between 2 mm and 4 mm.

6. The stopper of any of the preceding claims, wherein the third rib has a length between 0.5 mm and 1.5 mm.

7. The stopper of any of the preceding claims, wherein the fourth rib has a length between 0.5 mm and 1.5 mm.

8. The stopper of any of the preceding claims, wherein each of the first rib and fourth rib have a length greater than a length of either of the second rib or third rib.

9. The stopper of any of the preceding claims, wherein the second plurality of ribs is distally spaced from the first plurality ribs between 2 mm and 4 mm.

10. An injection device, comprising:
a syringe barrel; and
a stopper movably positioned within the syringe barrel, the stopper including:
a main body defining a proximal end, a distal end, and a sidewall extending between the proximal end and the distal end;
a first plurality of ribs adjacent the proximal end; and
a second plurality of ribs adjacent the distal end, wherein the second plurality of ribs is distally spaced from the first plurality ribs between 2 mm and 4 mm.

11. The injection device of claim 10, wherein at least one rib of the first plurality of ribs defines an interference level with respect to the syringe barrel between 3% and 15%.

12. The injection device of claims 10 or 11, wherein at least one rib of the second plurality of ribs defines an interference level with respect to the syringe barrel between 3% and 15%.

13. The injection device of any of claims 10-12, wherein each rib of the first plurality of ribs has substantially the same length.

14. The injection device of any of claims 10-13, wherein each rib of the second plurality of ribs has substantially the same length.

15. The injection device of any of claims 10-14, wherein at least one rib of the first plurality of ribs has a larger length than at least one other rib of the first plurality of ribs.

16. The injection device of any of claims 10-15, wherein at least one rib of the second plurality of ribs has a larger length than at least one other rib of the second plurality of ribs.
